# EUROPÄISCHE PATENTANMELDUNG

(11) **EP 2 923 759 A1**
(43) Veröffentlichungstag der Anmeldung: **30.09.2015**
(21) Anmeldenummer: 15156113.1
(22) Anmeldetag: 23.02.2015
(51) Int. Cl.: B01L 3/00, B04B 5/04, C12M 1/00, C12N 1/06

(54) **Vorrichtung zur automatisierten Prozessierung von biologischen Proben**

(30) Priorität: 27.03.2014 DE 102014205699
(71) Anmelder: ROBERT BOSCH GMBH, 70442 Stuttgart (DE)
(72) Erfinder: Hoehl, Melanie, 71254 Ditzingen (DE); Kehrbusch, Jenny, 66969 Lemberg (DE); Lemuth, Karin, 70195 Stuttgart (DE)

(57) **Zusammenfassung**

Bei einer gestapelten, mikrofluidischen Vorrichtung zur automatisierten Prozessierung von biologischen Proben weist die Vorrichtung Mikrostrukturen (110; 120) zum mechanischen Aufschluss von Zellen oder anderen Strukturen in der Probe auf.

## Beschreibung

Die vorliegende Erfindung betrifft eine Vorrichtung zur automatisierten Prozessierung von biologischen Proben sowie eine Einrichtung zum Einsetzen in eine Vorrichtung, die zur automatisierten Prozessierung von biologischen Proben vorgesehen ist.

### Stand der Technik

Die Aufbereitung und Prozessierung von biologischen Proben, beispielsweise im Zusammenhang mit der Aufreinigung und/oder Anreicherung bestimmter Moleküle oder mit der Analyse und Charakterisierung bestimmter Moleküle, basiert im Wesentlichen auf der Handhabung von Flüssigkeiten. In vielen Bereichen der Biologie und Biochemie und insbesondere auch in der medizinischen Forschung und Diagnostik wird eine Analyse von biologischem Zellmaterial durchgeführt. Beispielsweise werden Proteine und andere Bestandteile einer biologischen Zelle untersucht und charakterisiert.

Um die in einer Probe enthaltenen Proteine, Nukleinsäuren oder anderes Material untersuchen zu können, ist es oftmals erforderlich, die in der Probe enthaltenen biologischen Zellen aufzubrechen. Für das Aufbrechen der Zellen, das auch allgemein als Lyse bezeichnet wird, sind verschiedene Methoden bekannt. Hierbei müssen die Zellmembranen und im Fall von Bakterien, Pilzen und pflanzlichen Zellen auch die Zellwände zerstört werden. Beispielsweise ist eine enzymatische Lyse von Zellen sehr verbreitet, bei der Zellmembran- und Zellwandbestandteile enzymatisch abgebaut werden. Hierfür können beispielsweise die Enzyme Proteinase K oder Lysozym verwendet werden. Weiterhin können biologische Zellen auch chemisch oder mechanisch aufgeschlossen werden, beispielsweise durch eine Homogenisierung unter großem Druck (French-Press), durch Einsatz von Ultraschall, durch wiederholtes Einfrieren und Auftauen, durch elektromagnetische Strahlen oder durch Verwendung von sogenannten Kugelmühlen, bei denen eine Zerstörung der Zellen durch Scherkräfte erreicht wird, die bei dem "Mahlen" der Zellen zwischen kleinen Kügelchen (Beads) entstehen.

Insgesamt sollte ein Zellaufschluss schnell und effizient erfolgen. Das gewählte Verfahren sollte so ausgelegt sein, dass Zellmembranen und Zellwände auf der einen Seite zuverlässig zerstört werden. Auf der anderen Seite sollten die zellulären Bestandteile, die das jeweilige "Zielprodukt" des durchzuführenden Prozesses darstellen, nicht zerstört werden. Es sollte also beispielsweise keine weitergehende Denaturierung, Proteolyse oder Oxidation von Molekülen stattfinden.

Für viele biochemische Prozessierungen sind bereits Automatisierungen verfügbar, wobei beispielsweise Pipettierroboter oder andere Spezialgeräte zum Einsatz kommen. Weiterhin können mit sogenannten Lab-on-α-Chip-Systemen viele biochemische Prozesse in voll automatisierter Weise durchgeführt werden. Labon-α-Chip-Systeme sind mikrofluidische Systeme, die die gesamte Funktionalität eines makroskopischen Labors auf einem nur etwa westentaschengroßen Kunststoffsubstrat vereinigen.

Weiterhin sind kartuschenbasierte Systeme bekannt, wobei die Flüssigkeiten typischerweise in einem Spezialgerät in einer Kartusche prozessiert werden. Beispielsweise beschreibt die deutsche Offenlegungsschrift DE 10 2010 003 223 A1 ein System mit einer Vorrichtung, die zum Einsetzen in einen Zentrifugationsrotor vorgesehen ist. Hierbei sind zwei oder mehr revolverartige Körper axial übereinander angeordnet. Die Revolver beinhalten dabei ein oder mehrere Kavitäten, insbesondere Reaktionskammern und Kanäle, und gegebenenfalls weitere Strukturen für die Durchführung von Prozessen, insbesondere von fluidischen Einheitsoperationen. Ein Beschleunigungswechsel der Zentrifuge aktiviert eine integrierte Mechanik, die nach Art einer Kugelschreibermechanik funktioniert. Infolge der Zentrifugalkraft bewegen sich die Körper radial nach außen, wobei die Körper mittels einer Verzahnung und einem integrierten Rückstellmittel gegeneinander verschoben oder verdreht werden. Einzelne Kavitäten können hierdurch miteinander verschaltet werden. Darüber hinaus ist ein orientierungsabhängiges Öffnen von einzelnen Kavitäten oder Gefäßen in den Körpern möglich, wobei eine Seite der Kavität oder des Gefäßes beispielsweise mit einer durchstechbaren Folie versehen ist. Mit Hilfe eines Dorns auf einem anderen Körper wird die Folie durch die Bewegung der Körper gegeneinander durchstoßen. Auf diese Weise kann eine kontrollierte Fluidführung in der Vorrichtung erreicht werden. Beispielsweise kann eine Fluidführung von Vorlagerungskammern über zwischengeschaltete Prozessierungskammern bis hin zu Auffangkavitäten für die prozessierten Flüssigkeiten realisiert werden. Ein derartiges System kann zur Aufreinigung von biologischen oder biochemischen Molekülen genutzt werden. Hierfür werden im obersten Revolver die Probe und alle zur Aufreinigung benötigten Reagenzien eingesetzt. Die darunterliegenden Revolver dienen als Reaktionsstufen für die verschiedenen Fest- oder Flüssigphasenreaktionen. Der Transport von Probe und Reagenzien vom obersten zum untersten Revolver erfolgt durch die Zentrifugalkraft einer Standard-Laborzentrifuge, indem die Flüssigkeiten entlang des Kraftvektors der Zentrifugalkraft von radial innenliegenden Punkten zu radial außenliegenden Punkten transportiert werden.

### Offenbarung der Erfindung

### Vorteile der Erfindung

Die erfindungsgemäße Vorrichtung ist zur automatisierten Prozessierung von biologischen Proben geeignet, wobei innerhalb der Vorrichtung ein mechanischer Aufschluss von Zellen oder von anderen Strukturen in der biologischen Probe erfolgt. Mit dem hier verwendeten Begriff "Zellen" sind in erster Linie biologische Zellen und insbesondere prokaryontische und eukaryontische Zellen pflanzlichen oder tierischen oder menschlichen Ursprungs sowie auch Pilzzellen gemeint. Weiterhin ist hiermit auch Zellmaterial in Form von beispielsweise Geweben und/oder Organen gemeint. Darüber hinaus können mithilfe der erfindungsgemäßen Vorrichtung auch andere Strukturen wie beispielsweise Sporen oder andere Überdauerungsformen oder Erscheinungsformen von Lebewesen aufgeschlossen werden. Für den mechanischen Aufschluss dieser Zellen oder dieser anderen Strukturen sind erfindungsgemäß Mikrostrukturen in der erfindungsgemäßen Vorrichtung enthalten, insbesondere scharfkantige Mikrostrukturen. In besonders bevorzugter Weise handelt es sich bei diesen Mikrostrukturen um spitze oder scharfe Strukturen, vorzugsweise um Mikroklingen und/oder Mikromesser und/oder Mikrospitzen, die in die Vorrichtung eingebracht sind. Mittels dieser Strukturen können die in der biologischen Probe enthaltenen Zellen oder anderen Strukturen zuverlässig aufgeschlossen werden, um beispielsweise Nukleinsäuren oder Proteine aus den Zellen für eine Analyse oder für andere Zwecke zu gewinnen. Der Aufschluss erfolgt dabei schnell und effizient. Innerhalb der Vorrichtung zur automatisierten Prozessierung können die Zielmoleküle schnell von den Zelltrümmern abgetrennt werden, sodass die Zielmoleküle für weitere Schritte zur Verfügung stehen, ohne dass beispielsweise eine Denaturierung oder andere Schädigungen der Zielmoleküle erfolgen.

Der Kern der Erfindung liegt damit in dem mechanischen Zellaufschluss mittels der erfindungsgemäß vorgesehenen Mikrostrukturen. Die Zellen oder die anderen Strukturen werden mit diesen Mikrostrukturen in Kontakt gebracht und beispielsweise durch diese Strukturen gepresst. Die Zellen oder die anderen Strukturen werden dabei zerschnitten oder allgemein zerstört, sodass intrazelluläre Komponenten zugänglich werden. Dieser mechanische Zellaufschluss ist ohne Weiteres mit weiteren Prozessschritten und beispielsweise in Kombination mit einem komplexen Proteinaufreinigungs- oder DNA-Aufreinigungsprotokoll einsetzbar. Erfindungsgemäß befinden sich die Strukturen für den mechanischen Zellaufschluss innerhalb einer gestapelten mikrofluidischen Vorrichtung, die zur automatisierten Prozessierung vorgesehen ist.

Bei der erfindungsgemäßen Vorrichtung kann es sich insbesondere um eine Zentrifugationseinheit handeln, wobei die biologische Probe infolge der wirkenden Zentrifugalkraft durch die Vorrichtung geführt wird. Durch die wirkende Zentrifugalkraft können die Zellen oder die anderen Strukturen gegen oder durch die Mikrostrukturen gedrückt oder gepresst werden. Hierbei entsteht unter anderem eine Schneidwirkung der Mikrostrukturen. Diese Schneidwirkung ist besonders effektiv, wenn die Mikrostrukturen so orientiert sind, dass die Schneidwirkung entsteht, wenn die Probe infolge die Zentrifugalkraft von oben nach unten durch die Vorrichtung gedrückt wird. In anderen Ausführungsformen kann die erfindungsgemäße Vorrichtung mittels Druck und/oder Vakuum prozessiert werden.

In bevorzugter Weise weist die erfindungsgemäße mikrofluidische Vorrichtung wenigstens zwei axial übereinander angeordnete (gestapelte) Körper auf, die jeweils wenigstens eine Kavität enthalten. Die Körper sind in Abhängigkeit von einer Zentrifugalkraft oder einer gleich wirkenden Kraft (z.B. Druck oder Vakuum) gegeneinander verschiebbar oder verdrehbar, wobei die Kavitäten der übereinander angeordneten Körper miteinander fluidisch koppelbar sind. Die Körper sind beispielsweise als sogenannte Revolver ausgestaltet. Die erfindungsgemäße Vorrichtung umfasst neben den wenigstens zwei axial übereinander angeordneten (gestapelten) Körpern ein Gehäuse, das die gestapelten Körper aufnimmt. Das Gehäuse ist insbesondere so ausgestaltet, dass es in die Halterung eines Rotors einer Zentrifuge eingesetzt werden kann. Das Gehäuse kann beispielsweise die Form eines üblichen Zentrifugenröhrchens aufweisen. Der Transport von Flüssigkeiten durch die übereinander angeordneten Körper erfolgt in vorgebbarer Weise durch Ausnutzung der Zentrifugalkraft oder einer gleichwirkenden Kraft. Insbesondere handelt es sich um eine mikrofluidische Anordnung, wie sie beispielsweise aus der eingangs bereits erwähnten deutschen Offenlegungsschrift DE 10 2010 003 223 A1 bekannt ist. Für die automatisierte Prozessierung von biologischen Proben in einem solchen System ist es bereits bekannt, die Zellen mittels Enzymen aufzuschließen oder zu lysieren. Dies hat allerdings den Nachteil, dass der Vorgang des Zellaufschlusses verhältnismäßig lange dauert. Zudem sind die erforderlichen Enzyme und Reagenzien teuer und eine Lagerung eines solchen Systems mit den erforderlichen Enzymen muss im Allgemeinen bei niedrigen Temperaturen erfolgen, damit die Enzyme ihre Aktivität nicht verlieren. Für die eigentliche Durchführung des Zellaufschlusses ist oftmals eine höhere Temperatur erforderlich, da die Enzyme in der Regel ein bestimmtes Temperaturoptimum für ihre maximale Aktivität aufweisen. Demgegenüber nutzt die erfindungsgemäße Vorrichtung einen mechanischen Zellaufschluss unter Verwendung von Mikrostrukturen. Es sind hierbei keine teuren Reagenzien, insbesondere keine teuren Enzyme, erforderlich. Auch eine Lagerung einer derartigen Vorrichtung ist im Gegensatz zur Lagerung einer Vorrichtung mit enthaltenen Enzymen ganz unproblematisch, da beispielsweise keine Lagerung bei niedrigen Temperaturen oder beispielsweise bei bestimmten Pufferbedingungen erforderlich ist. Auch der Zellaufschluss selbst ist weitestgehend unabhängig von der Temperatur, sodass für den Zellaufschluss keine bestimmte Temperatur eingestellt werden muss. Der mechanische Zellaufschluss ist sehr schnell durchführbar, beispielsweise in weniger als fünf Minuten. Insgesamt erlaubt die erfindungsgemäße Vorrichtung mit dem mechanischen Aufschluss der Zellen oder der anderen Strukturen im Vergleich mit einem enzymatischen Zellaufschluss eine wesentlich schnellere, einfachere und kostengünstigere Durchführung des Zellaufschlusses.

In bevorzugter Weise können die Mikrostrukturen als vorstehende Mikroklingen, Mikrospitzen oder Mikromesser ausgestaltet sein. Die Höhe der Mikrostrukturen kann hierbei beispielsweise in einem Bereich zwischen circa 50 nm und 1 mm, insbesondere zwischen circa 50 nm und 500 µm liegen, vorzugsweise zwischen circa 100 nm und 50 µm. Der Abstand zwischen einzelnen Mikrostrukturen, insbesondere zwischen den Spitzen der Mikrostrukturen, kann beispielsweise in einem Bereich zwischen circa 40 nm und 1 mm liegen, insbesondere zwischen circa 80 nm und 650 µm, bevorzugt zwischen circa 500 nm und 40 µm, besonders bevorzugt zwischen circa 500 nm und 1 µm. Die Wahl der Abmessungen der jeweils eingesetzten Mikrostrukturen kann zweckmäßigerweise an die Größe der aufzuschließenden Zellen angepasst werden. Besonders vorteilhaft ist es, wenn der Abstand zwischen den einzelnen Mikrostrukturen kleiner als der Durchmesser der aufzuschließenden Zellen ist. Die genannten Abmessungen sind beispielsweise für Zellen geeignet, die einen Durchmesser zwischen etwa 200 nm und 50 µm aufweisen. Für *Escherichia coli* ist beispielsweise ein Abstand zwischen den Mikrostrukturen von 40 nm bis 1 µm und insbesondere von 40 nm bis 500 nm besonders geeignet. Die Abmessungen können auch abweichend und insbesondere größer als die genannten Werte sein. Die genannten Angaben sind insbesondere für die Abmessungen von Mikronadeln oder Mikroklingen geeignet.

In einer Ausgestaltung der erfindungsgemäßen Vorrichtung sind im Bereich der Mikrostrukturen und insbesondere unterhalb der Mikrostrukturen ein oder mehrere Kanäle zum Weiterleiten der aufgeschlossenen Zellen oder der aufgeschlossenen anderen Strukturen vorgesehen. In dieser Ausgestaltung kann das aufgeschlossene Material direkt in eine nachfolgende Reaktionskammer (Kavität) zur weiteren Prozessierung weitergeleitet werden.

In einer besonders bevorzugten Ausgestaltung der Vorrichtung sind zwischen und/oder unterhalb der Mikrostrukturen Kanäle vorgesehen, deren Durchmesser kleiner als der Abstand zwischen den einzelnen Mikrostrukturen ist. Beispielsweise beträgt der Durchmesser der Kanäle zwischen circa 10 nm und 10 µm. In dieser Ausführungsform können die Zellen und anschließend die aufgeschlossenen Zellen (Zelllysat) in gewisser Weise durch die Mikrostrukturen hindurchgepresst werden. Durch die hierbei entstehenden Scherkräfte kann der Aufschluss der Zellen oder der anderen Strukturen noch weiter gefördert werden.

Die erfindungsgemäßen Mikrostrukturen können an unterschiedlicher Position oder an unterschiedlichen Positionen innerhalb der Vorrichtung angeordnet sein. Besonders bevorzugt ist es, wenn die Mikrostrukturen in einer Kavität der Vorrichtung vorgesehen sind, die zum Auftragen der Probe vorgesehen ist, sodass die Zellen oder die anderen Strukturen gleich zu Beginn des automatisierten Prozesses aufgeschlossen werden. Es kann jedoch auch vorgesehen sein, dass alternativ oder zusätzlich die erfindungsgemäßen Mikrostrukturen in einer anderen Kavität vorgesehen sind, beispielsweise in einer Kavität, die als Mischkammer dient.

In einer bevorzugten Ausgestaltung der erfindungsgemäßen Vorrichtung sind die Mikrostrukturen in Form von ineinander greifenden Mikrostrukturen, insbesondere in Form von ineinander greifenden Spitzen und/oder Zähnen ausgebildet. Hierbei kann die Vorrichtung beispielsweise einen Körper beziehungsweise Revolver umfassen, der eine Mischwanne und einen Mischeinsatz enthält. Diese beiden Elemente sind beispielsweise durch einen federbasierten Mechanismus gegeneinander beweglich. Durch wiederholte Änderung der Zentrifugalkraftbeschleunigung kommt es zu einer Auf- und Abwärtsbewegung der ineinander gesetzten Teile und damit zu einer Mischung der dazwischen befindlichen Flüssigkeiten. Eine solche an sich bekannte Mischeinrichtung wird erfindungsgemäß so umgestaltet, dass zum einen auf dem Boden der Mischwanne und zum anderen auf der Unterseite des Mischeinsatzes erfindungsgemäß Mikrostrukturen angeordnet werden. Diese Mikrostrukturen sind vorzugsweise in Form von ineinandergreifenden Zähnen oder Spitzen ausgebildet. Wenn sich im Zuge der Zentrifugalkraftbeschleunigungsänderungen die Mischwanne und der Mischeinsatz gegeneinander bewegen, greifen die Mikrostrukturen vergleichbar mit einem Beißmechanismus wiederholt ineinander, sodass dazwischenliegende Zellen oder Strukturen aus der biologischen Probe zerstört beziehungsweise aufgeschlossen werden. Die ineinandergreifenden Zähne oder Spitzen können beispielsweise eckig (kubisch) oder gezackt, z.B. in Form von pyramidalen oder kegelförmigen Spitzen ausgestaltet sein. Die Höhe der Zähne oder Spitzen kann hierbei beispielsweise in einem Bereich zwischen circa 100 nm und 10 mm, insbesondere zwischen circa 100 nm und 1 mm liegen, vorzugsweise zwischen circa 500 nm und 500 µm. Die Breite der Zähne oder Spitzen kann beispielsweise in einem Bereich zwischen circa 100 nm und 10 mm liegen, insbesondere zwischen circa 100 nm und 100 µm, vorzugsweise zwischen circa 100 nm und 10 µm, insbesondere zwischen circa 100 nm und 5 µm. Der Abstand zwischen einzelnen Zähnen oder Spitzen kann beispielsweise in einem Bereich zwischen circa 10 nm und 50 µm liegen, insbesondere zwischen circa 40 nm und 1 µm, vorzugsweise zwischen circa 40 nm und 500 nm. Bei den pyramidalen Zähnen bezieht sich die Angabe der Breite der Zähne insbesondere auf eine Breite an der Basis der Zähne.

Die erfindungsgemäßen Mikrostrukturen können auf verschiedene Weise hergestellt werden, beispielsweise durch eine Strukturierung von Oberflächen. Die Mikrostrukturen können auch als separate Strukturen auf eine Unterlage aufgebracht werden. Die Mikrostrukturen können beispielsweise aus Keramik und/oder einem dielektrischen Material und/oder aus Glas und/oder aus Metall und/oder aus einem Halbleitermaterial und/oder aus Silizium und/oder aus Polymeren gefertigt sein.

Es kann vorgesehen sein, dass die Mikrostrukturen als Einlegeteil in eine Vorrichtung zur automatisierten Prozessierung von biologischen Proben eingebracht werden. Die Erfindung umfasst daher auch eine Einrichtung zum Einsetzen in eine Vorrichtung zur automatisierten Prozessierung von biologischen Proben, wobei die Einrichtung Mikrostrukturen, insbesondere scharfkantige Mikrostrukturen, zum mechanischen Aufschluss von Zellen oder anderen Strukturen in der Probe aufweist. Bei diesen Mikrostrukturen kann es sich beispielsweise um Mikroklingen oder Mikromesser oder Mikrospitzen handeln. Wenn in der Vorrichtung ein oder mehrere Kanäle zur Weiterleitung des Zelllysates vorgesehen sind, sollte der Durchmesser der Kanäle zweckmäßigerweise nicht größer als das Einlegeteil sein. Bezüglich weiterer Merkmale der Mikrostrukturen einer solchen Einrichtung beziehungsweise eines solchen Einlegeteils wird auf die obige Beschreibung verwiesen.

Schließlich umfasst die Erfindung ein Verfahren zur automatisierten Prozessierung von biologischen Proben, wobei eine Vorrichtung oder eine Einrichtung (Einlegeteil) für eine solche Vorrichtung, wie sie oben beschrieben sind, verwendet wird. Mit diesem Verfahren ist ein besonders vorteilhafter Aufschluss der Zellen oder der anderen Strukturen aus der biologischen Probe möglich, da der Zellaufschluss in automatisierter Weise sehr schnell und kostengünstig erfolgen kann. Es sind keine teuren Reagenzien erforderlich, die unter Umständen bei niedrigen Temperaturen gelagert werden müssten. Zudem muss der Zellaufschluss selbst nicht bei einer bestimmten Temperatur, insbesondere nicht bei einer erhöhten Temperatur, durchgeführt werden.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

### Kurze Beschreibung der Zeichnungen

In den Zeichnungen zeigen:
- Fig. 1: schematische Darstellung einer zentrifugierbaren Vorrichtung mit mehreren zueinander verdrehbaren Körpern (Revolver) aus dem Stand der Technik;
- Fig. 2A/B: isometrische Darstellungen eines Körpers aus einer zentrifugierbaren Vorrichtung mit erfindungsgemäßen Mikrostrukturen;
- Fig. 3A/B: isometrische Darstellungen eines Körpers aus einer zentrifugierbaren Vorrichtung mit erfindungsgemäßen Mikrostrukturen;
- Fig. 4: isometrische Darstellung einer Mischeinrichtung mit Mischwanne und Mischeinsatz als Bestandteil einer zentrifugierbaren Vorrichtung;
- Fig. 5A/B: isometrische Ansichten der Mischwanne und des Mischeinsatzes aus Figur 4 mit Positionierung der erfindungsgemäßen Mikrostrukturen;
- Fig. 6A/B: beispielhafte Ausgestaltungen der erfindungsgemäßen Mikrostrukturen in schematischer Darstellung und
- Fig. 7: schematische Darstellung der erfindungsgemäßen Mikrostrukturen mit Kanälen zum Abführen des Zelllysats.

### Beschreibung von Ausführungsbeispielen

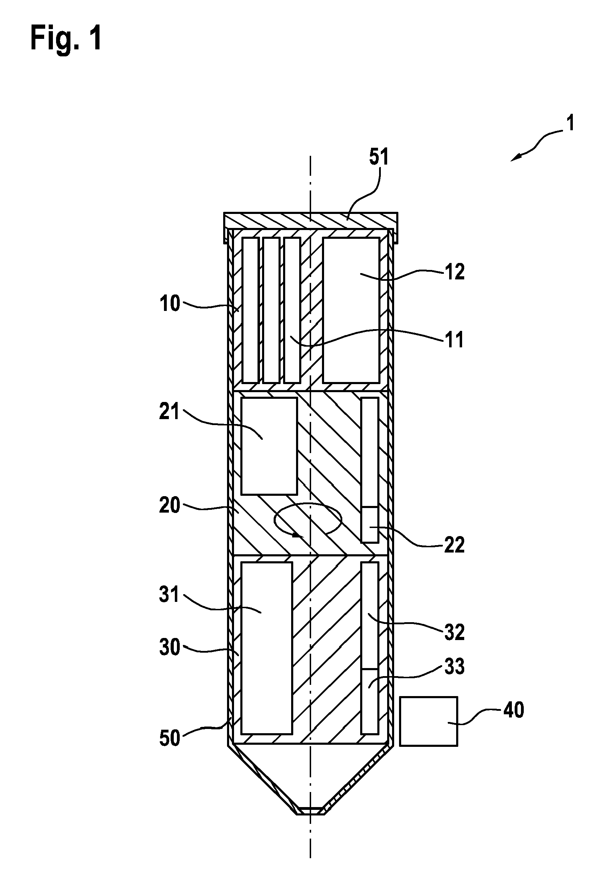

Fig. 1 zeigt in schematischer Weise ein System 1 zur automatisierten Prozessierung von biologischen Proben aus dem Stand der Technik, das auf mehreren axial übereinander angeordneten Körpern (Revolvern) 10, 20, 30 beruht, die gegeneinander verdrehbar sind. Die Körper 10, 20, 30 umfassen verschiedene Kavitäten 11, 12, 21, 22, 31, 32, 33, die als Behältnisse und/oder Reaktionsräume dienen. Die Vorrichtung kann beispielsweise für eine Proteinaufreinigung oder DNA-Aufreinigung eingesetzt werden. In den Kavitäten 11 werden Reagenzien vorgehalten. In die Kavität 12 wird die Probe eingebracht. Die Kavität 21 stellt eine Mischkammer dar. Die Kavität 22 enthält eine Matrixbasierte Säule, mit der die eigentliche Protein- oder DNA-Aufreinigung erfolgt. Die Kavität 31 ist für den Abfall aus Waschschritten an der Säule vorgesehen. In der Kavität 32 wird das Eluat der Säule aufgefangen. In einer anschließenden Reaktionskammer 33 kann das aufgereinigte Protein oder die DNA mit einer biochemischen Reaktion nachgewiesen werden, wobei ein Detektor 40, der sich außerhalb der Vorrichtung befindet, eingesetzt werden kann. Die Körper 10, 20, 30 befinden sich in gestapelter Weise innerhalb eines Gehäuses in Form eines Zentrifugenröhrchens 50, das mit einem Deckel 51 verschließbar ist. Das Zentrifugenröhrchen 50 wird in den Rotor einer Laborzentrifuge eingesetzt. Durch die wirkenden Zentrifugalkräfte verdrehen sich die Körper 10, 20, 30 in vorgegebener Weise gegeneinander, insbesondere über eine integrierte Kugelschreibermechanik. Der Transport von Probe und Reagenzien vom obersten Revolver 10 bis zum untersten Revolver 30 erfolgt durch die Zentrifugalkraft. Hierbei kann es vorgesehen sein, dass durch Dorne oder ähnliches an den Körpern 20, 30 die darüberliegenden Kavitäten geöffnet werden. Der Fluidfluss erfolgt in vorgegebener Weise, sodass die Probe aus der Kavität 12 in vorbestimmter Weise die verschiedenen Prozessierungsschritte durchläuft.

Um mit einer solchen Vorrichtung beispielsweise ein intrazelluläres Protein aufreinigen zu können, müssen die Zellen, die dieses Protein exprimieren, zunächst aufgeschlossen werden. Herkömmlicherweise kann dies beispielsweise vorab erfolgen, indem die Zellen außerhalb der Vorrichtung beispielsweise enzymatisch oder chemisch behandelt werden, sodass das resultierende Zelllysat dann in die Kavität 12 eingebracht werden kann, um weiter prozessiert zu werden. Eine andere herkömmliche Möglichkeit ist, dass in einer solchen Vorrichtung ein Enzym, das zum Lysieren der Zellen geeignet ist, vorgehalten wird. Das Enzym kann beispielsweise in der Kavität 12 enthalten sein. Wenn dann die Probe, die die Zellen enthält, in die Kavität 12 eingebracht wird, findet unter bestimmten Bedingungen eine Zelllyse, d.h. also ein Zellaufschluss, statt. Hierbei ist allerdings zu beachten, dass das Temperaturoptimum des jeweiligen Enzyms zu berücksichtigen ist. In der Regel ist eine oberhalb der Raumtemperatur liegende Temperatur für die enzymatische Aktivität erforderlich. Weiterhin ist zu beachten, dass die Stabilität des Enzyms bei der Lagerung einer derartigen Vorrichtung zu berücksichtigen ist, wobei in der Regel eine Stabilität des Enzyms nur bei ausreichend tiefen Temperaturen gewährleistet ist. Um diese verschiedenen Probleme und Nachteile zu vermeiden, sieht die erfindungsgemäße Vorrichtung vor, eine derartige Vorrichtung mit Mikrostrukturen, insbesondere mit Mikroklingen, Mikromessern und/oder Mikrospitzen auszustatten, um einen mechanischen Zellaufschluss durchführen zu können. Mithilfe dieser Mikrostrukturen kann der Zellaufschluss sehr einfach, unkompliziert und schnell sowie in automatisierter Weise durchgeführt werden. Vorzugsweise sind zwischen oder unter den Mikrostrukturen Durchlässe vorgesehen, sodass das Zelllysat automatisch für eine weitere Prozessierung weitergeleitet werden kann. Es sind keine kostenintensiven Enzyme erforderlich. Weiterhin müssen keine bestimmten Temperaturen zum einen für die Lagerung von Enzymen und zum anderen für deren optimale enzymatische Aktivität berücksichtigt werden.

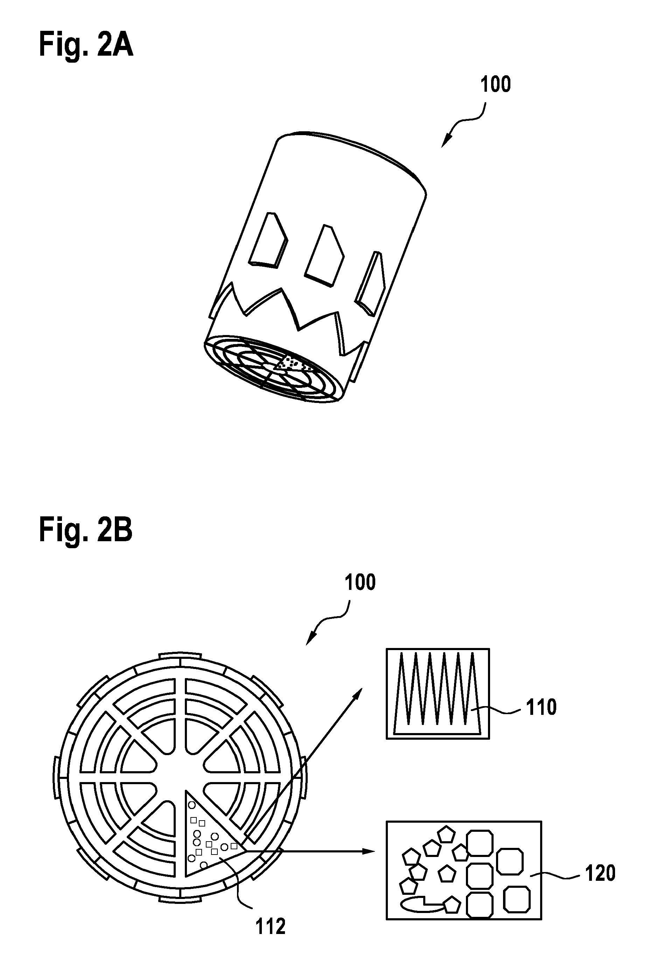

**Fig. 2A** zeigt eine Seitenansicht eines Körpers oder eines Revolvers 100, der im Zusammenhang mit der beschriebenen Zentrifugationseinheit aus Fig. 1 eingesetzt werden kann. Der Revolver 100 kann beispielsweise als oberster Revolver in der Zentrifugationseinheit 1 aus Fig. 1 eingesetzt werden. Im Inneren weist der Revolver 100 mehrere Kavitäten auf, die zum Vorhalten von Reagenzien und zum Probenauftrag vorgesehen sind. **Fig. 2B** zeigt den Revolver 100 in Aufsicht. Die Kavität 112 ist als Probenauftragskammer vorgesehen. Die Kammer 112 ist mit den erfindungsgemäßen Mikrostrukturen ausgestattet, die zum Aufschluss der in der Probe enthaltenen Zellen oder anderen Strukturen vorgesehen sind. Gezeigt sind zwei Beispiele für mögliche Ausgestaltungen der Mikrostrukturen. Beispiel 110 zeigt Mikronadeln oder Mikroklingen. Beispiel 120 zeigt Mikrostrukturen in Form von Mikroscherben. Die Mikronadeln oder Mikroklingen 110 können durch eine Oberflächenstrukturierung in der Kammer 112 in einfacher Weise, beispielsweise durch 3D-Druck, realisiert werden. Eine andere Möglichkeit ist das Einsetzen eines Einlegeteils, das entsprechende Mikrostrukturen aufweist. Als Materialien für die Herstellung der Mikrostrukturen eignen sich im Prinzip alle mikrostrukturierbaren Materialien wie zum Beispiel Keramiken, Dielektrika, Glas, Metall, Halbleiter, Silizium oder Polymere. Die Mikrostrukturen können beispielsweise aus 3D-mikrodruckbaren Polymeren (3D-Laserlithografie) oder aus Metallen nach einer Strukturinversion hergestellt werden.

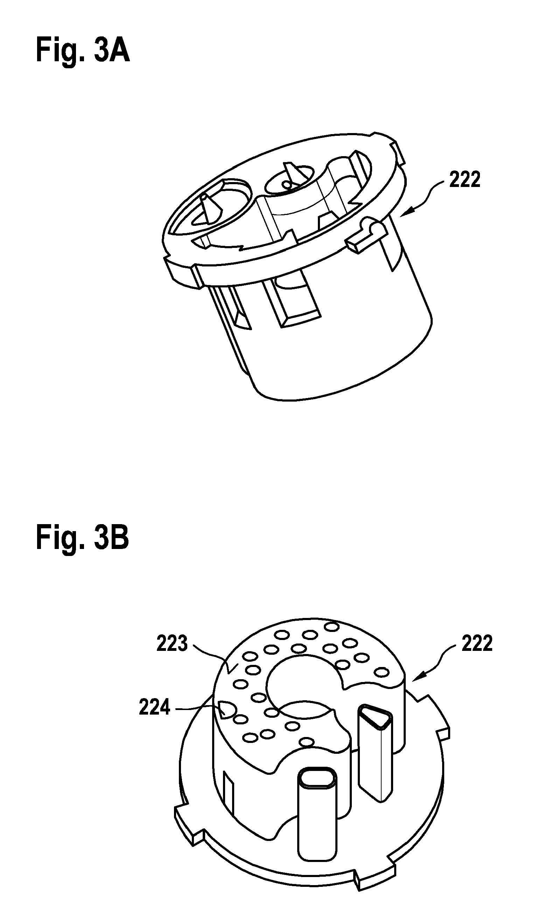

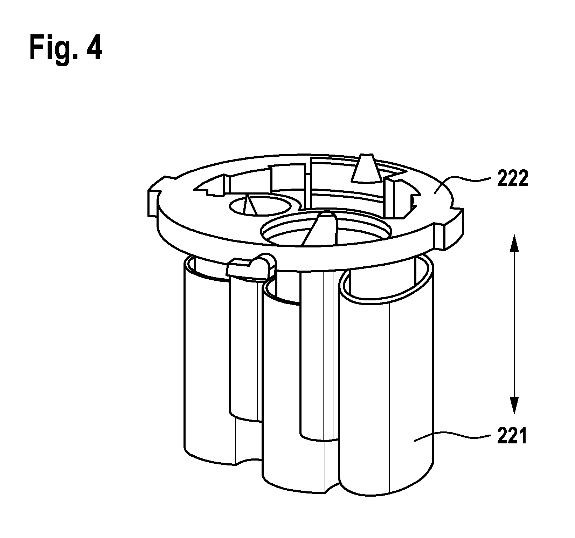

Eine alternative oder zusätzliche Anordnung von Mikrostrukturen ist in **Fig. 3A****/B** illustriert. Fig. 3 zeigt einen Mischeinsatz 222 in zwei Ansichten. Dieses Element kann beispielsweise Teil des mittleren Revolvers 20 der in Fig. 1 illustrierten Zentrifugationseinheit 1 sein. Der äußere Boden 223 des Mischeinsatzes 220 ist erfindungsgemäß mit Mikrostrukturen ausgestattet, beispielsweise mit Mikronadeln, Mikroklingen oder Mikroscherben, hier angedeutet als Kreise. Mithilfe dieser Mikrostrukturen werden die in der Mischwanne 221 enthaltenen Zellen in der biologischen Probe zerstört und aufgeschlossen. Im Boden des Mischeinsatzes 222 ist eine Öffnung 224 zum Durchtritt von Flüssigkeiten vorgesehen. Dies wird anhand der Fig. 4 näher erläutert.

**Fig. 4** illustriert die Verwendung des Mischeinsatzes 222 im Zusammenspiel mit einer Mischwanne 221. Der Mischeinsatz 222 greift in das Innere der Mischwanne 221 ein und ist durch eine Feder gekoppelt. Durch wiederholte Änderung der Zentrifugalbeschleunigung, beispielsweise zwischen 20 und 14.000 x g, kommt es zu einer Auf- und Abwärtsbewegung der ineinandergesetzten Teile, die hier durch den Doppelpfeil (Zentrifugalkraft vs. Federkraft) angedeutet ist. Auf diese Weise wird eine Mischung der in der Mischwanne 221 und dem Mischeinsatz 222 enthaltenen Flüssigkeiten in an sich bekannter Weise erreicht, wobei die Flüssigkeiten durch eine Öffnung im Boden des Mischeinsatzes 222 während der Auf- und Abwärtsbewegungen hindurchfließen können.

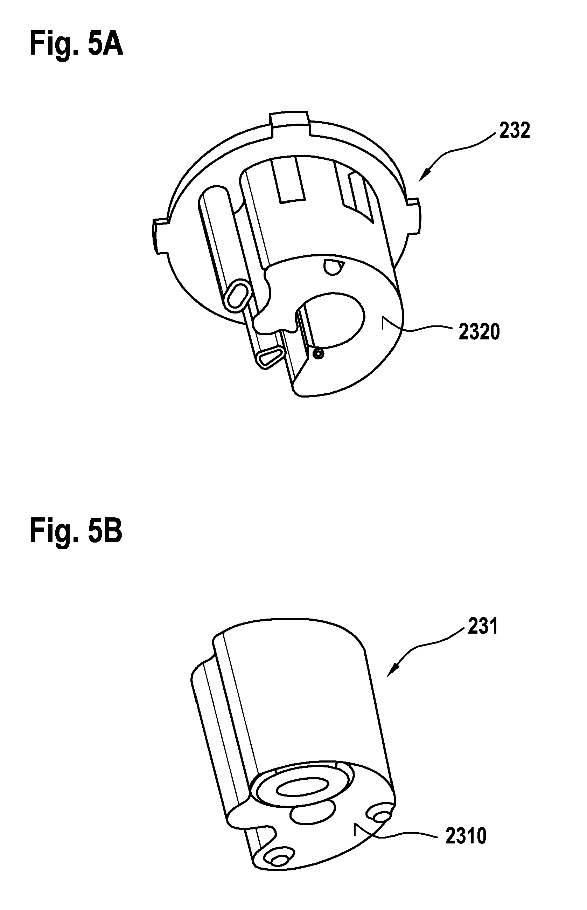

**Fig. 5A****/B** illustriert eine weitere erfindungsgemäße Ausgestaltung der Mischwanne 231 und des Mischeinsatzes 232 mit Mikrostrukturen gemäß der Erfindung. Hierbei ist einerseits der Boden des Mischeinsatzes 232 auf der unteren Seite 2320 mit Mikrostrukturen versehen (nicht näher dargestellt). Andererseits ist der Boden 2310 der Mischwanne 231 auf der Innenseite ebenfalls mit Mikrostrukturen versehen. Diese Mikrostrukturen sind in der Form von ineinandergreifenden Zähnen oder Nadeln ausgestaltet. Wenn die Unterseite des Mischeinsatzes 232 auf die Innenseite der Mischwanne 231 im Zuge der Auf- und Abwärtsbewegung der ineinandergesetzten Teile gepresst wird, werden die in der Probe enthaltenen Zellen oder die anderen Strukturen bei dem Ineinandergreifen der Nadeln oder Zähne zerstört und aufgeschlossen.

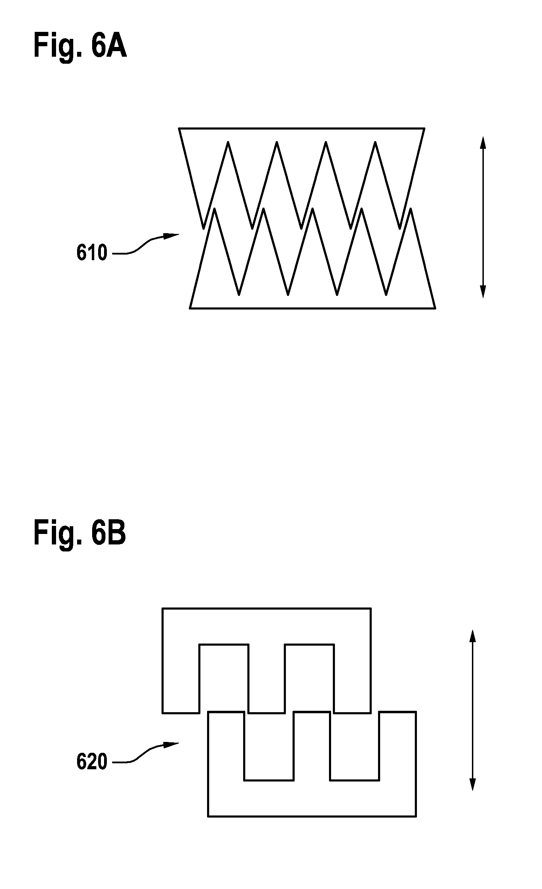

**Fig. 6A****/B** illustriert in schematischer Weise (nicht maßstabsgetreu) zwei mögliche Ausgestaltungen der ineinander greifenden Zähne der Ausführungsform aus Fig. 5. Fig. 6A zeigt zackenförmige, ineinander greifende Zähne (Spitzen) 610. Fig. 6B zeigt eckig ausgeformte, ineinander greifende Zähne 620, wobei in beiden Beispielen gewissermaßen ein Beißmechanismus bei der Auf- und Abwärtsbewegung der ineinander gesetzten Mischwanne und des Mischeinsatzes realisiert wird. Die Höhe der Zähne oder Spitzen kann beispielsweise zwischen circa 100 nm und 10 mm, insbesondere zwischen circa 1 µm und 2 mm liegen. Die Breite der Zähne oder Spitzen kann beispielsweise zwischen circa 100 nm und 10 mm, vorzugsweise zwischen circa 1 µm und 2 mm betragen.

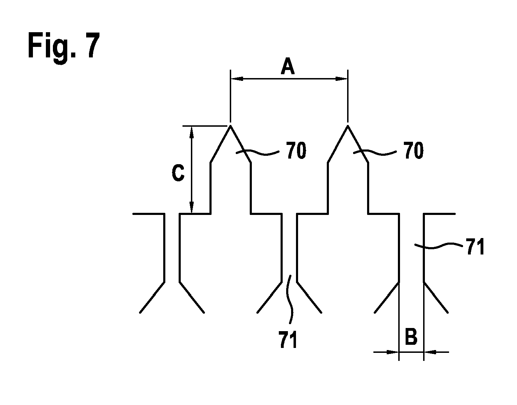

**Fig. 7** zeigt ein weiteres Beispiel für die Ausgestaltung der erfindungsgemäßen scharfkantigen Mikrostrukturen. In dieser Ausgestaltung sind die Mikrostrukturen als Mikronadeln oder Mikroklingen 70 realisiert. Zwischen bzw. unterhalb der einzelnen Mikronadeln oder Mikroklingen 70 sind Kanäle 71 angeordnet. Die Kanäle 71 dienen zum Abführen der aufgeschlossenen Probe, also des Zelllysats. Hierbei ist es vorteilhaft, wenn der Abstand A zwischen den einzelnen Mikronadeln oder Mikroklingen 70 größer ist als der Durchmesser B der Kanäle 71. Der Abstand A zwischen den Spitzen der einzelnen Mikronadeln oder Mikroklingen kann beispielsweise zwischen 40 nm und 1 mm liegen. Der Durchmesser B der Kanäle 71 kann beispielsweise zwischen 10 nm und 10 µm liegen. Die Höhe C der Mikrostrukturen kann beispielsweise zwischen circa 50 nm und 1 mm liegen. Diese Abmessungen und Verhältnisse werden zweckmäßigerweise an die aufzuschließenden Zellen oder Strukturen angepasst. Insbesondere sollte der Abstand A kleiner als der Durchmesser der zu lysierenden Zellen sein. Daher können für besonders große Zellen die angegebenen Werte auch überschritten werden. Größere Abmessungen sind beispielsweise auch für die Verarbeitung von Gewebeproben geeignet, die mithilfe der erfindungsgemäßen Vorrichtung zerkleinert und aufgeschlossen werden können.

Im Folgenden werden einige Zentrifugationsprotokolle erläutert, die bei der Verwendung einer Zentrifugationseinheit, die mit den Mikrostrukturen gemäß der Erfindung ausgestattet ist, eingesetzt werden können. Bei der Zentrifugationseinheit handelt es sich insbesondere um ein System mit drei übereinander gestapelten Revolvern, wie es in schematischer Weise anhand von Figur 1 erläutert wurde.

Das erste Beispiel bezieht sich auf einen Zellaufschluss im obersten Revolver. Hierbei ist beispielsweise der Boden einer Probenauftragskammer im obersten Revolver mit Mikronadeln ausgestattet. Die Probe mit den enthaltenen Zellen wird in die Probenkammer aufgegeben. Im Zuge des durchzuführenden Zentrifugationsprotokolles wird die Probe durch den Mikronadelboden hindurchgeschoben, wobei die Zellen zerstört und aufgeschlossen werden. Das hierfür eingesetzte Zentrifugenprotokoll sieht vor, dass die Zentrifuge zunächst auf 1.000 bis 6.000 x g beschleunigt wird, insbesondere auf 6.000 x g. Diese Beschleunigung wird für eine Sekunde bis eine Minute gehalten. Anschließend wird auf 200 x g oder weniger, vorzugsweise auf 40 x g, herunterbeschleunigt. Hierbei wird die in der Zentrifugationseinheit integrierte Kugelschreibermechanik aktuiert, wodurch ein Aufstechmechanismus ausgelöst wird, der eine Folie auf der Unterseite der Probenauftragskammer durchsticht und die lysierte Probe in eine darunterliegende Kavität entlässt. Diese Schritte sowie die anschließenden Schritte zur Durchführung einer säulenchromatographischen Reinigung von Proteinen oder DNA an einer Säulenmatrix sind in der Tabelle 1 dargestellt, wobei im Allgemeinen ein Lastwechsel zwischen einer Beschleunigung von 600 x g oder mehr auf der einen Seite und 200 x g oder weniger, vorzugsweise 40 x g, auf der anderen Seite für eine Betätigung der Schaltmechanik eingesetzt wird.

**Tabelle 1**

| **Prozessschritt** | **Zentrifugalbeschleunigung (g)** | **Zyklus-Zahl** |
|---|---|---|
| Lyse in Revolver 1 | 1000-6000 g, 1s-1min | 1 |
| Freilassen des Lysats zu Revolver 2 und Kugelschreibermechanik | 40 g, 30s | 1 |
| Anstechen des Revolvers 1, um das lysierte Probenmaterial mit z.B. Bindepuffer zu mischen | 600 g für 30s und 40 g für 30s | 1 |
| Mischen | 500 g, 5s; 5000 g, 5s | 20 |
| Binden auf die Säule | 600 g für 30s und 40 g für 30s | 1 |
| Freisetzen Waschpuffer 1 und 2 | 600 g für 30s und 40 g für 30s | 2 |
| Trockenschleudern der Säule | 6000 g | 5min |
| Freisetzen Elutionsflüssigkeit aus Revolver 1 | 600 g für 30s und 40 g für 30s | 1 |
| Elution | 6000 g | 3min |

In einem weiteren Beispiel erfolgt der Zellaufschluss durch die erfindungsgemäß vorgesehenen Mikrostrukturen innerhalb des mittleren Revolvers, wobei die Mikrostrukturen in die Mischkammer der Zentrifugationseinheit integriert sind. Hierbei können die Mikrostrukturen, insbesondere die Mikromesser oder Mikronadeln, auf der Außenseite des Bodens eines Mischeinsatzes angeordnet sein, wie es anhand von Figur 3A/B erläutert wurde. Weiterhin können auch auf der Innenseite der Mischwanne Mikrostrukturen vorgesehen sein, sodass die jeweiligen Mikrostrukturen bei einer Auf- und Abwärtsbewegung der ineinandergreifenden Mischwanne und des Mischeinsatzes nach Art eines Beißmechanismus ineinandergreifen, wie es anhand von Figur 5A/B und Figur 6A/B bereits erläutert wurde. Durch das Gegeneinanderschieben von Mischwanne und Mischeinsatz, insbesondere über ein federbetriebenes System, werden turbulente Strömungen und dadurch Scherkräfte erzeugt, die herkömmlicherweise für eine Durchmischung der Flüssigkeiten ausgenutzt werden. Erfindungsgemäß werden die Mikrostrukturen an dem Mischeinsatz und/oder der Mischwanne genutzt, um während des Durchschiebens der Flüssigkeiten durch die Mischvorrichtung einen Zellaufschluss zu erreichen. Das Mischen kann beispielsweise durch ein Zyklieren zwischen den Beschleunigungszahlen im Bereich von 100 und 5.500 x g oder 500 und 5000 x g stattfinden. Ein solches Zyklieren der Beschleunigungen kann beispielsweise mit einer Zykluszahl zwischen 1 und 500 durchgeführt werden. Besonders geeignet sind Zykluszahlen zwischen 20 und 200. Ein beispielhaftes Zentrifugationsprotokoll für einen Zellaufschluss und eine anschließende DNA-Aufreinigung ist der nachfolgenden Tabelle 2 zu entnehmen.

**Tabelle 2**

| **Prozessschritt** | **Zentrifugalbeschleunigung (g)** | **Zyklus-Zahl** |
|---|---|---|
| Anstechen Revolver 1 (Probe wird in die Mischkammer in Revolver 2 gebracht) | 600 g für 30s und 40 g für 30s | 1 |
| Mischen in Mischkammer in Revolver 2 inklusive Lyse | 500 g, 5s; 5000 g, 5s | 100 |
| Anstechen des Revolvers 1, um das lysierte Probenmaterial mit z.B. Bindepuffer zu mischen | 600 g für 30s und 40 g für 30s | 1 |
| Mischen | 500 g, 5s; 5000 g, 5s | 20 |
| Binden auf die Säule | 600 g für 30s und 40g für 30s | 1 |
| Freisetzen Waschpuffer 1 und 2 | 600 g für 30s und 40 g für 30s | 2 |
| Trockenschleudern der Säule | 600 g | 5min |
| Freisetzen Elutionsflüssigkeit aus Revolver 1 | 600 g für 30s und 40 g für 30s | 1 |
| Elution | 6000g | 3min |

Durch den Einsatz der erfindungsgemäßen Mikrostrukturen erfolgt im Allgemeinen keine Zerstörung der doppelsträngigen DNA, deren Abmessungen im Bereich unter 5 nm liegen, also weit unter dem Zelldurchmesser. Im Zuge des erfindungsgemäßen Zellaufschlusses bleiben die DNA und andere Moleküle durch die umgebenden Zelltrümmer geschützt. Der DNA-Strang kann zwar möglicherweise fragmentiert werden, aber es bleiben in jedem Fall ausreichende Fragmente für eine weitere molekularbiologische Verarbeitung oder Analyse intakt.

## Patentansprüche

1. Vorrichtung zur automatisierten Prozessierung von biologischen Proben, **dadurch gekennzeichnet, dass** die Vorrichtung eine gestapelte, mikrofluidische Vorrichtung ist, die Mikrostrukturen (110; 120; 610; 620; 70) zum mechanischen Aufschluss von Zellen oder anderen Strukturen in der Probe aufweist.

2. Vorrichtung nach Anspruch 1, **dadurch gekennzeichnet, dass** die Mikrostrukturen (110; 120; 610; 620; 70) Mikroklingen und/oder Mikromesser und/oder Mikrospitzen sind.

3. Vorrichtung nach Anspruch 1 oder Anspruch 2, **dadurch gekennzeichnet, dass** die Vorrichtung eine Zentrifugationseinheit ist.

4. Vorrichtung nach einem der vorhergehenden Ansprüche, **dadurch gekennzeichnet, dass** die Vorrichtung für eine Prozessierung mittels Druck und/oder Vakuum vorgesehen ist.

5. Vorrichtung nach einem der vorhergehenden Ansprüche, **dadurch gekennzeichnet, dass** die Vorrichtung wenigstens zwei axial übereinander angeordnete Körper (10; 20; 30) mit jeweils wenigstens einer Kavität (11, 12, 21, 22, 31, 32, 33) aufweist und wobei die Körper in Abhängigkeit von einer Zentrifugalkraft oder einer gleichwirkenden Kraft gegeneinander verschiebbar oder verdrehbar sind und wobei die Kavitäten miteinander fluidisch koppelbar sind.

6. Vorrichtung nach einem der vorhergehenden Ansprüche, **dadurch gekennzeichnet, dass**
- die Höhe der Mikrostrukturen (110; 120; 70) zwischen circa 50 nm und 1 mm, vorzugsweise zwischen circa 50 nm und 500 µm, noch bevorzugter zwischen circa 100 nm und 50 µm, beträgt und/oder
- der Abstand zwischen einzelnen Mikrostrukturen (110; 120; 70) zwischen circa 40 nm und 1 mm, insbesondere zwischen circa 80 nm und 650 µm, vorzugsweise zwischen circa 500 nm und 40 µm, noch bevorzugter zwischen circa 500 nm und 1 µm, beträgt,
wobei vorzugsweise die Abmessungen der Mikrostrukturen in Abhängigkeit von den aufzuschließenden Zellen gewählt werden.

7. Vorrichtung nach einem der vorhergehenden Ansprüche, **dadurch gekennzeichnet, dass** im Bereich der Mikrostrukturen (70) Kanäle (71) zum Weiterleiten der aufgeschlossenen Zellen oder der aufgeschlossenen anderen Strukturen vorgesehen sind, wobei vorzugsweise der Durchmesser der Kanäle kleiner als der Abstand zwischen einzelnen Mikrostrukturen ist und wobei vorzugsweise der Durchmesser der Kanäle (71) zwischen circa 10 nm und 10 µm beträgt.

8. Vorrichtung nach einem der Ansprüche 4 bis 7, **dadurch gekennzeichnet**, das die Mikrostrukturen (110; 120) in einer Kavität (112) enthalten sind, die zum Auftragen der Probe vorgesehen ist und/oder dass die Mikrostrukturen in einer Kavität (231, 232) enthalten sind, die als Mischkammer vorgesehen ist.

9. Vorrichtung nach einem der vorhergehenden Ansprüche, **dadurch gekennzeichnet, dass** die Mikrostrukturen in Form von ineinander greifenden Mikrostrukturen, insbesondere in Form von ineinander greifenden Spitzen (610) oder Zähnen (620) ausgebildet sind.

10. Vorrichtung nach Anspruch 9, wobei die Vorrichtung eine Mischwanne (231) und einen Mischeinsatz (232) aufweist, die gegeneinander beweglich sind, **dadurch gekennzeichnet, dass** die Mikrostrukturen (610; 620) einerseits auf dem Boden (2310) der Mischwanne und andererseits auf der Unterseite (2320) des Mischeinsatzes angeordnet sind.

11. Vorrichtung nach Anspruch 9 oder Anspruch 10, **dadurch gekennzeichnet, dass**
- die Höhe der Mikrostrukturen (610; 620) zwischen circa 100 nm und 10 mm, vorzugsweise zwischen circa 100 nm und 1 mm, noch bevorzugter zwischen circa 500 nm und 500 µm, beträgt und/oder
- die Breite der Mikrostrukturen (610; 620) zwischen circa 100 nm und 10 mm, insbesondere zwischen circa 100 nm und 100 µm, vorzugsweise zwischen circa 100 nm und 10 µm, noch bevorzugter zwischen circa 100 nm und 5 µm, beträgt und/oder
- der Abstand zwischen einzelnen Mikrostrukturen (610; 620) zwischen circa 10 nm und 50 µm, vorzugsweise zwischen circa 40 nm und 1 µm, beträgt.

12. Vorrichtung nach einem der vorhergehenden Ansprüche, **dadurch gekennzeichnet, dass** die Mikrostrukturen (110; 120; 610; 620; 70) aus Keramik und/oder einem dielektrikischen Material und/oder Glas und/oder Metall und/oder einem Halbleitermaterial und/oder Silizium und/oder Polymeren gefertigt sind.

13. Einrichtung zum Einsetzen in eine Vorrichtung (1) zur automatisierten Prozessierung von biologischen Proben, **dadurch gekennzeichnet, dass** die Einrichtung Mikrostrukturen (110; 120; 610; 620; 70) zum mechanischen Aufschluss von Zellen oder anderen Strukturen in der Probe aufweist.

14. Einrichtung nach Anspruch 13, weiter **gekennzeichnet durch** wenigstens eines der Merkmale gemäß einem der Ansprüche 2 bis 12.

15. Verfahren zur automatisierten Prozessierung von biologischen Proben, **dadurch gekennzeichnet, dass** eine Vorrichtung gemäß einem der Ansprüche 1 bis 12 und/oder eine Einrichtung gemäß Anspruch 13 oder Anspruch 14 zum mechanischen Aufschluss von Zellen oder anderen Strukturen in der Probe verwendet wird.
